(19) Europäisches Patentamt
European Patent Office
Office européen des brevets

(11) **EP 1 641 444 B1**

(12) **EUROPEAN PATENT SPECIFICATION**

(45) Date of publication and mention
of the grant of the patent:
**23.03.2016 Bulletin 2016/12**

(51) Int Cl.:
*A61K 31/12* (2006.01)  *A61K 31/455* (2006.01)
*A61K 31/19* (2006.01)  *A61K 31/216* (2006.01)
*A61P 21/00* (2006.01)  *A61K 31/70* (2006.01)

(21) Application number: **04735029.3**

(22) Date of filing: **27.05.2004**

(86) International application number:
**PCT/GB2004/002286**

(87) International publication number:
**WO 2004/105742 (09.12.2004 Gazette 2004/50)**

(54) **TREATMENT OF MUSCLE FATIGUE**

BEHANDLUNG VON MUSKELERMÜDUNG

TRAITEMENT DE FATIGUE MUSCULAIRE

(84) Designated Contracting States:
**AT BE BG CH CY CZ DE DK EE ES FI FR GB GR
HU IE IT LI LU MC NL PL PT RO SE SI SK TR**

(30) Priority: **02.06.2003 GB 0312603
13.06.2003 GB 0313760**

(43) Date of publication of application:
**05.04.2006 Bulletin 2006/14**

(73) Proprietor: **ISIS INNOVATION LIMITED
Summertown,
Oxford OX2 7SG (GB)**

(72) Inventors:
• **CLARKE, Kieran
Summertown
Oxford OC2 7BZ (GB)**
• **SCHEUERMANN-FREESTONE, Michaela
Summertown
Oxford OC2 7BZ (GB)**
• **MURRAY, Andrew, James
Summertown
Oxford OC2 7BZ (GB)**

(74) Representative: **Geary, Stephen et al
Bawden & Associates
4 The Gatehouse
2 High Street
Harpenden, Herts AL5 2TH (GB)**

(56) References cited:
**WO-A-01/51645          DE-U- 20 205 184
US-A1- 2001 047 008     US-A1- 2004 063 661**

**US-B1- 6 544 960**

• SIMONS, L.A. ET AL: "Long-term treatment with slow release oxprenolol alone or in combination with other drugs: Effects on blood pressure, lipoproteins and exercise performance" AUST. N.Z. J. MED., vol. 12, 1982, pages 612-616, XP009037249
• EAGLES C J ET AL: "THE EFFECTS OF COMBINED TREATMENT WITH BETA,-SELECTIVE RECEPTOR ANTAGONIST AND LIPID-LOWERING DRUGS ON FAT METABOLISM AND MEASURES OF FATIGUE DURING MODERATE INTENSITY EXERCISE: A PLACEBO-CONTROLLEDSTUDY IN HEALTHY SUBJECTS" BRITISH JOURNAL OF CLINICAL PHARMACOLOGY, BLACKWELL SCIENTIFIC PUBL, GB, vol. 43, 1997, pages 291-300, XP002941319 ISSN: 0306-5251
• PATENT ABSTRACTS OF JAPAN vol. 1998, no. 11, 30 September 1998 (1998-09-30) & JP 10 175855 A (TAISHO PHARMACEUT CO LTD), 30 June 1998 (1998-06-30)
• PATENT ABSTRACTS OF JAPAN vol. 0163, no. 58 (C-0970), 4 August 1992 (1992-08-04) & JP 4 112825 A (RIKAGAKU KENKYUSHO; others: 01), 14 April 1992 (1992-04-14)

**(Cont. next page)**

- **KOHUT,M.L. ET AL.: "Effects of decreased free fatty acids on fatigue during exercise with carbohydrate feeding" MEDICINE AND SCIENCE IN SPORTS AND EXERCISE - 42 ANNUAL MEETING OF THE AMERICAN COLLEGE OF SPORTS MEDICINE MINNEAPOLIS, MINNESOTA, USA MAY 31-JUNE 3 1995, vol. 27, no. 5 suppl, 1995, page S102, XP009037250**
- **DATABASE WPI Derwent Publications Ltd., London, GB; AN 2004-401576 XP002298605 & CN 1 483 355 A ((GAOL-I) GAO) 24 March 2004 (2004-03-24)**

Remarks:
The file contains technical information submitted after the application was filed and not included in this specification

**Description**

Field of the Invention

This invention relates to the treatment of muscle impairment or fatigue, in particular to the treatment of cardiovascular disease and in particular heart failure.

Background of the Invention

Cardiovascular disease is the leading cause of death in patients with type 2 diabetes,[1] who have decreased survival after myocardial infarction and increased congestive heart failure and silent ischemia compared with non-diabetic control subjects.[1,2] Type 2 diabetes mellitus is a chronic metabolic disorder characterised by insulin resistance, hyperglycemia, hyperinsulinemia and elevated plasma free fatty acids, with poor glycemic control associated with an increased risk of heart failure.[2,3] Therapeutic interventions that normalise glucose and lipid metabolism reduce the incidence of cardiovascular disease in patients with diabetes,[4] with metabolic control of diabetes being the most important predictor of cardiovascular morbidity and mortality.[1]

In the normal adult heart, free fatty acids, glucose and lactate are metabolised for ATP production in the mitochondria. However, in the diabetic heart, glucose and lactate oxidation are decreased[5,6] and fatty acid oxidation is increased,[7] increasing the oxygen requirement perATP molecule produced.[2,4,7,8] Positron emission tomographic studies have shown that patients with type 2 diabetes have decreased resting myocardial blood flow rates[9] and decreased fluorodeoxyglucose uptake rates,[10] yet little is known of cardiac high energy phosphate metabolism in these patients. Similarly, skeletal muscle blood flow,[11] and glucose transmembrane transport and oxidation[2] are decreased in diabetes. Patients with type 2 diabetes have limited exercise tolerance,[2,12,13] which has been associated with decreased glycemic control[12] and microvascular disease.[13]

WO00/64876 discloses tri-aryl acid derivatives that modulate the function of peroxisome proliferator-activated receptors (PPAR), to decrease triglyceride levels and therefore treat disorders associated with high levels of triglyceride, including diabetes.

WO01/74834 discloses specific compounds that inhibit sodium-dependent glucose transporters and can therefore be used for treating diabetes and associated complications.

WO02/028857 discloses specific compounds that can act as antidiabetics. The compounds are stated to have multiple activities including the reduction of plasma triglycerides and free-fatty acids.

WO99/24451 discloses adenosine derivatives that inhibit lipolysis and therefore decrease free fatty acid levels.

WO01/51645 discloses polypeptides that decrease free-fatty acid levels, with the purpose of decreasing body mass.

Although the prior art discloses that reduction of free-fatty acid levels is desirable in the treatment or prevention of diabetes and its associated complications, there is no mention that such a reduction in free-fatty acid levels can be used to treat muscle fatigue or impairment.

Summary of the Invention

The present invention is based in part on the finding that cardiac high energy phosphate metabolism is significantly altered in patients with type 2 diabetes, despite apparently normal cardiac morphology and function. The alteration in phosphate metabolism correlates with circulating free fatty acid and glucose concentrations. In contrast, skeletal muscle energetics and oxygenation are normal at rest, but deoxygenation and loss of phosphocreatine are faster during exercise and reoxygenation and phosphocreatine recovery are slower following exercise. These findings suggest that alterations in cardiac and skeletal muscle energetics occur early in the pathophysiology of type 2 diabetes and are associated with alterations in metabolic substrates. The findings suggest that lowering free fatty acids will be useful in the treatment of muscle impairment generally, in particular cardiac muscle impairment. Furthermore, the reduction of free fatty acids may be a desirable aim in the treatment of disorders associated with mitochondrial dysfunction. The treatment of cardiac muscle impairment is distinct from the treatment of cardiovascular disease, which is caused by the build up of arthereosclerotic plaques in the vasculature; the present invention, by contrast, involves the repair (or prevention of damage to) the cardiac muscle.

According to a first aspect of the invention, a compound that reduces the level of free fatty acids circulating in the plasma of a subject is used in the treatment or prevention of muscle (particularly cardiac or skeletal muscle) impairment or fatigue, the compound comprising a ketone body ester.

The compound can be used in patients suffering in particular any of the following conditions : diabetes, cardiac impairment, hypopyrexia, hyperthyroidism, metabolic syndrome X, fever and infection.

The compound may be administered in any suitable form and by any suitable route of administration. In one embodiment, the compound may be administered in a food or drink supplement.

In a preferred embodiment, the ketone body ester induces mild ketosis.

According to a second aspect of the invention a liquid composition for rehydration during or after exercise comprises water, a sugar carbohydrate and a compound that reduces free fatty acids circulating in the blood plasma being a ketone body ester.

Description of the Drawings

The present invention is described with reference to the accompanying drawings, wherein:

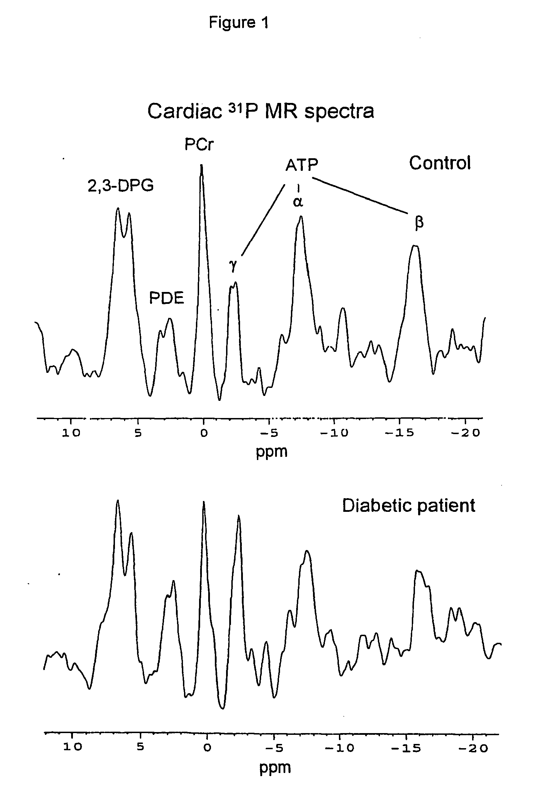

Figure 1 shows a typical cardiac 31p MR spectra from a normal control (upper spectrum) and a patient with type 2 diabetes (lower spectrum), showing the lower PCr/ATP ratio in the patient; 2,3-DGP, (2, 3-diphosphoglycerate) PDE (phosphodiesters) PCr (phosphocreatine) a,, and y indicate the three phosphate groups of ATP;

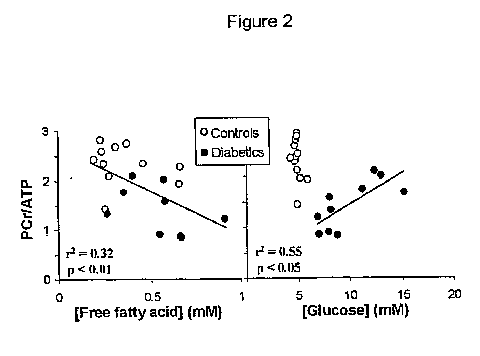

Figure 2 is a graph showing high energy phosphate levels, expressed as the PCr to ATP ratio (Pcr/ATP) ; trendlines are shown to guide the eye;

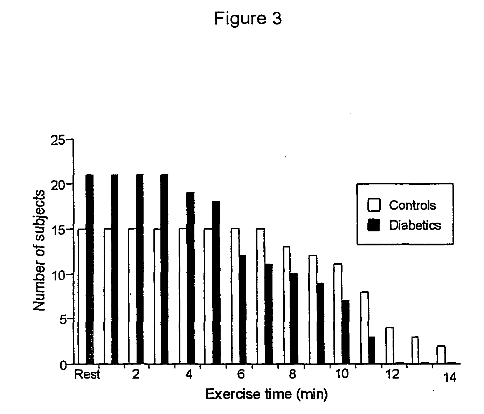

Figure 3 is a graph showing skeletal muscle exercise tolerance, expressed as exercise time, in patients with type 2 diabetes (n=21) and control subjects (n=15) ; the number of subjects exercising is plotted for each minute of exercise, showing that the patients were unable to exercise for as long as the controls ;

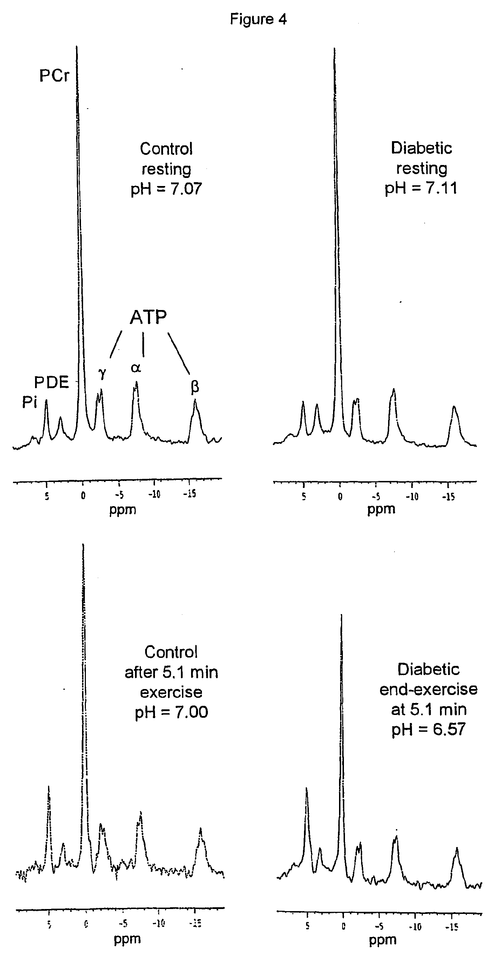

Figure 4 is a graph showing typical calf muscle $^{31}$P MR spectra from a control subject and a patient with type 2 diabetes at rest (upper panel, number of scans=64), from the same patient at the end of exercise and the same matched control at the equivalent time (5.1 min) of exercise (lower panel, number of scans=16); Pi (inorganic phosphate) PDE (phosphodiesters) PCr (phosphocreatine) $\alpha$, $\beta$ and $\gamma$ indicate the three phosphate groups of ATP; the cytosolic pH was calculated from the chemical shift of Pi relative to PCr; the abscissa shows the chemical shift in parts per million (ppm); and

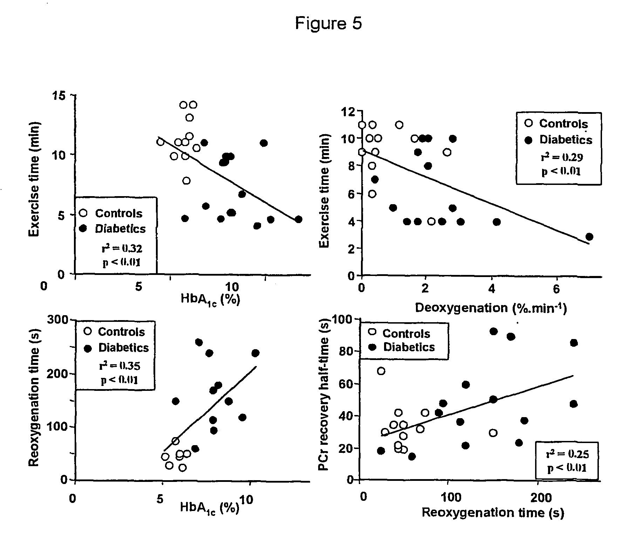

Figure 5 shows exercise times correlated with HbA1c levels and with skeletal muscle deoxygenation rates during exercise in patients with type 2 diabetes (n=14) and control subjects (n=12); trendlines are shown to guide the eye.

Description of the Invention

The term "PCr" used herein refers to phosphocreatine; the term "PDE" refers to phosphodesters; and the term "ATP" refers to adenosine triphosphate, as will be appreciated by the skilled person.

The present invention shows that high energy phosphate metabolism is significantly impaired in cardiac and skeletal muscle in patients with type 2 diabetes who have apparently normal cardiac morphology and function. The PCr/ATP ratios correlated negatively with the circulating free fatty acids in all subjects tested and positively with the plasma glucose in patients with diabetes. Furthermore, faster skeletal muscle PCr loss is found together with pH decline and deoxygenation during exercise in patients with diabetes and slower PCr recovery following exercise; the PCr recovery half-times correlate with the reoxygenation times for all subjects.

**Cardiac metabolism**

Hyperinsulinemia, hyperglycemia and increased lipid and lipoprotein abnormalities associated with type 2 diabetes may negatively influence myocardial performance,[4] but, in the early stages of diabetes mellitus, systolic function is often preserved despite changes in cardiac substrate metabolism.[5,6] It is unknown whether substrate changes in diabetes mellitus[2,4-7] alter myocardial high energy phosphate metabolism. $^{31}$P MRS is the only non-invasive tool for measurement of high energy phosphate metabolism in the human heart, although limited to measurement of the PCr/ATP ratio in routine patient studies. Despite the limited information obtainable from human heart, compared with $^{31}$P MRS of isolated heart[6] and skeletal muscle,[15] a $^{31}$P MRS study of patients with dilated cardiomyopathy has shown a low cardiac PCr/ATP ratio to be a strong predictor of total and cardiovascular mortality, superior to the measurement of ejection fraction.[18] The studies of the present invention revealed that the myocardial PCr/ATP ratio was 35% lower in type 2 diabetic patients, who had normal cardiac function, than in healthy controls. The PCr/ATP ratio correlated negatively with the plasma free fatty acid concentrations in all subjects because free fatty acid concentrations are not under tight metabolic control (Figure 2). Increased fatty acid availability results in increased free fatty acid uptake and oxidation in the mitochondria,[2,7] and increased expression of mitochondrial uncoupling proteins,[19] both of which decrease the amount of ATP produced per molecule of oxygen consumed in the mitochondrial electron transport chain.[2,19] Therefore the diabetic heart has an increased requirement for oxygen.[8]

The hyperglycemia that occurs with diabetes is known to compensate for the impaired capacity for myocardial glucose transport.[7] Glucose uptake is important for glycolytic ATP production during ischemia, low glucose uptake increasing ischemic injury in the heart.[6] Patients with type 2 diabetes have decreased fluorodeoxyglucose uptake rates,[10]

decreased resting myocardial blood flows[9] and an increased incidence of silent ischemia.[1] In the study detailed below, the lower cardiac PCr/ATP ratios in the patients who had lower plasma glucose concentrations suggested that decreased glucose availability may have limited glucose uptake. Although plasma lactate levels were 40% higher in the diabetic patients, and lactate is a metabolic substrate for the heart, the lack of a correlation between lactate levels and the cardiac PCr/ATP ratio was possibly because lactate oxidation is inhibited more than glucose oxidation in the diabetic heart.[20]

**Skeletal muscle metabolism**

Although cardiac high energy phosphate metabolism was abnormal in the patients with diabetes, the results of the study found that skeletal muscle energetics, pH and oxygenation were normal at rest. All subjects fatigued after the same tissue deoxygenation and with the same loss of PCr, increase in free ADP and at the same acidic pH. This suggests that substrate availability or metabolism and glycogen levels were not limiting the skeletal muscle energetic changes. Additionally, faster loss of PCr and decrease in pH during exercise with slower PCr recovery was found after exercise in the patients with diabetes. The PCr recovery half-times correlated with the plasma $HbA_{1c}$ and glucose, but not with fatty acid or lactate concentrations. However, deoxygenation was faster during exercise in the patients with diabetes and reoxygenation was slower following exercise and correlated with the PCr recovery half-times, suggesting that tissue oxygen availability was limiting ATP production. Elevated levels of $HbA_{1c}$ have been associated with microvascular complications[3,21,22] and reduced exercise capacity.[12] In the study indicated below, the exercise times and the reoxygenation times correlated with the $HbA_{1c}$ levels, indicating that abnormal skeletal muscle oxygenation in the patients with diabetes may have been related to microvascular disease. In diabetic patients with intermittent claudication, skeletal muscle reoxygenation took ~4 times longer than in normal subjects and provided a more sensitive measure of lower leg claudication than ankle pressure measurements.[23] Consequently, microvascular disease may explain most, if not all, of the abnormalities in skeletal muscle high energy phosphate metabolism that were observed in patients with diabetes.

The following is a non-exhaustive list of conditions that are associated with high levels of free fatty acids. Patients with the following disorders may benefit from treatment with compounds that reduce the levels of free fatty acids: Neurodegenerative diseases including , but not limited to, Alzheimer's disease, Parkinson's disease, Huntington's chorea; hypoxic states including, but not limited to, angina pectoris, extreme physical exertion, intermittent claudication, hypoxia, stroke, myocardial infarction; insulin resistant states including, but not limited to, infection, stress, obesity, diabetes, heart failure; and inflammatory states including, but not limited to, infection, autoimmune disease.

Further studies demonstrated a positive correlation between circulating free fatty acids (FFAs) and the levels of the mitochondrial uncoupling proteins UCP-2 ($r^2$=0.42;P<0.001) and UCP-3 ($r^2$=0.22; P<0.05) but no correlation was found with any other plasma metabolite. This suggests that increased circulating FFA in humans increase UCP expression and thereby decrease cardiac efficiency. Reducing circulating FFA levels in patients may therefore provide a new treatment for heart failure that increases the efficiency of cardiac hydraulic work.

The following is a non-exhaustive list of conditions that are associated with mitochondrial dysfunction. Patients with the following disorders may also benefit from treatment with compounds that reduce the levels of free fatty acids in the blood:

Essential hypertension
Cardiomyopathy
Congenital muscular dystrophy
Immune (Hyper Thyroid)
Fatigue & Exercise intolerance
Hypertension
Kidney disease
Longevity (Aging)
MELASL (mitochondrial Encephalomopathy, Lactic Acidosis, and Stroke-Like episodes)
Deafness
Multiple symmetric lipomatosis
Myalgias
Myoglobinuria
Myopathy syndromes
Neoplasms (Cancer)
Optic atrophy
Rhabdomyolysis:mtDNA
Sudden infant death (SIDS)
Wilson's disease

The person skilled in the art will recognise that there are many compounds that are known to reduce fatty acid levels in blood plasma. For example, the compounds disclosed in the international (PCT) patent publications WO-A-00/64876, WO-A-01/74834 and WO-A-02/028857 may be used in combination with the ketone body ester in the present invention. Suitable compounds result in decreased free fatty acid levels of at least 5%, more preferably at least 20%, and most preferably at least 30%.

The medicament may be prepared in any convenient formulation, for oral, mucosal, pulmonary, intravenous or other delivery form. Suitable amounts will be apparent to the person skilled in the art, depending on the severity of the condition to be treated, age and weight of the patient, as will be appreciated by the skilled person.

In one aspect of the invention, there is a composition for rehydration during or after exercise, the composition comprising water, a sugar carbohydrate and a compound, being a ketone body ester, that reduces the levels of free fatty acids in the plasma of a patient. The composition may also comprise suitable flavourings, colourants and preservatives, as will be appreciated by the skilled person. The carbohydrate sugar is present as an energy source, and suitable sugars are known, including glucose and trehalose.

The ketone body ester may be used in a method for the treatment or prevention of muscle, particularly cardiac or skeletal muscle, impairment or fatigue. The method is carried out by administering a ketone body ester to a subject.

The compound will usually be administered in an amount to achieve a circulating concentration of from 10nm to 2um, preferably from 100nm to 500nm.

Measurement of free fatty acids can be accomplished by methods known in the art, and disclosed herein.

The following example illustrates the invention with reference to the accompanying figures.

**Example**

**Subjects and protocol**

Patients with type 2 diabetes (n=21) aged between 18 and 75 years with no evidence of cardiovascular disease or ECG-detectable evidence of ischemia were included in this study. Five patients were diet-controlled only, 6 patients each were treated with either a sulfonylurea drug or metformin, and 4 patients were treated with metformin and a sulfonylurea. Patients on insulin therapy were excluded. Patients were matched for age, sex and body mass index with healthy control subjects (n=15).

All procedures were conducted on the same day, at the same time of day, for each subject. Subjects were fasted overnight for 12 h before blood sampling and echocardiography. After a small breakfast, the cardiac (rest) and skeletal muscle (exercise) magnetic resonance spectroscopy (MRS) protocols were performed and the subjects had lunch. For the near-infrared spectrophotometry (NIRS) measurements of muscle oxygenation, the MRS exercise protocol was repeated outside the magnet because the NIRS probe was magnetic. The MRS and NIRS exercise bouts were separated by two hours of ambulatory rest and 30 minutes of supine rest, to ensure that all variables were stable.

**Blood tests and echocardiography**

Fasting blood was taken to determine glucose and glycosylated hemoglobin (HbA$_1$c) levels, lipid profiles and free fatty acid levels (Wako NEFA C enzyme assay, Wako Chemicals, Neuss, Germany). Because our magnetic resonance (MR) scanner was capable of MR spectroscopy, but not of precise left ventricular function analysis, we assessed cardiac function using a SONOS 5500 echocardiography machine (Hewlett Packard, Bracknell, UK). Left ventricular dimensions and mass index were obtained using M-mode echocardiography, and ejection fraction was calculated from left ventricular volumes, derived using the modified Simpson's rule. Diastolic function (early flow velocity (E) and late atrial contraction (A); E:A) was evaluated by acquisition of a pulsed Doppler recording trace through the mitral valve, with the sample volume positioned just above the mitral valve leaflet tips.

**Measurements of cardiac muscle metabolism**

Cardiac high energy phosphate metabolism was measured using $^{31}$P MRS on a 2 Tesla whole-body magnet (Oxford Magnet Technology, Eynsham, UK) which was interfaced to a Bruker Avance spectrometer (Bruker Medical GmbH, Ettlingen, Germany). Cardiac $^{31}$P MRS was performed with the subject in the prone position, as previously described.[14] Briefly, subjects were positioned with their heart at the isocentre of the magnet, which was confirmed using standard multislice spin-echo proton ($^1$H) images acquired with a double-rectangular surface coil placed around the chest (relaxation time TR=heart rate, echo time TE=25 ms, slice =10 mm, 15 mm spacing). Once the position was verified, the coil was exchanged for a circular proton surface coil (diameter 15 cm), and shimming was performed to optimise the magnetic field homogeneity over the heart. Finally, a $^{31}$P surface coil (diameter 8 cm) was used to acquire cardiac spectra using a slice-selective, one-dimensional chemical shift imaging (1 D-CSI) sequence, including spatial presaturation of lateral muscle (Figure 1). An 8 cm thick transverse slice was then excited, followed by one-dimensional phase encoding into the chest to subdivide signals into 64 coronal layers, each 1 cm thick (TR=heart rate, 16 averages). All $^1$H and $^{31}$P spectral acquisitions were cardiac gated and saturation corrected.[14] Spectra were Fourier-transformed, and a 15 Hz line broadening was applied. Spectra were fitted using a purpose-designed interactive frequency domain-fitting program. After fitting, the ATP peak area was corrected for blood contamination according to the amplitude of the 2,3-diphosphoglycerate (2,3-DPG) peak and the phosphocreatine (PCr)/ATP and phosphodiester (PDE)/ATP ratios were calculated and corrected for saturation as described earlier.[14] The chemical shift differences between the $\alpha$-and $\beta$-phosphate peaks of ATP were used as a measure of intracellular free magnesium concentrations.

**Measurements of skeletal muscle metabolism**

$^{31}$P MRS of the right gastrocnemius muscle was performed using the 2 Tesla magnet (see above) with the subject in a supine position and a 6 cm diameter surface coil under the muscle, as previously described.[15] Spectra were acquired using a 2 s interpulse delay at rest (64 scans/spectrum) and during exercise and recovery (16 scans/spectrum).[15] The muscle was exercised by plantar flexion against a standardised weight (10% lean body mass) at 0.5 Hz through a distance of 7 cm, with subsequent further increases of weight (2% of lean body mass every minute), and subjects were exercised until fatigued. Relative concentrations of inorganic phosphate, PCr and ATP were obtained using a time-domain fitting routine (VARPRO, R. de Beer, Delft, Netherlands) and were corrected for partial saturation. Absolute concentrations were obtained assuming that the concentration of cytosolic ATP was 8.2 mmol.l$^{-1}$ intracellular water and intracellular pH was calculated from the chemical shift of the Pi peak relative to PCr ($\delta$Pi; measured in parts per million, ppm), using the equation:

$$pH = 6.75 + \log\frac{\delta Pi - 3.27}{5.69 - \delta Pi}$$

The chemical shift differences between the $\alpha$- and $\beta$-phosphate peaks of ATP were used as a measure of intracellular free magnesium concentrations. Free cytosolic [ADP] was calculated from pH and [PCr] using a creatine kinase equilibrium constant[16] ($K_{ck}$) of 1.66 x 10$^9$.M$^{-1}$ and assuming a normal total creatine content of 42.5 mmol.l$^{-1}$, using the equation:

$$[ADP] = \frac{[ATP][total\ creatine]}{[PCr][H^+]K_{ck}}$$

At the end of exercise, because glycogenolysis had stopped and PCr resynthesis was purely oxidative, analysis of PCr recovery provided information about mitochondrial function. Recovery half-times for PCr and ADP, and initial rates of PCr recovery, were calculated as previously described.[15]

**Measurements of skeletal muscle oxygenation**

Muscle oxygen saturation (SmO$_2$) was measured using dual-wavelength NIRS (INVOS 4100 Oximeter, Somanetics, Troy, USA), with the light emittor and two sensors placed over the medial head of the right gastrocnemius muscle.[17] SmO$_2$ was determined using the ratio of absorbance at the wavelengths of 733 nm and 809 nm, which estimated deoxygenated and the sum of deoxygenated and oxygenated hemoglobin, respectively. SmO$_2$ was measured in deep tissue, predominantly at a depth of 2 cm, this being dependent on differentiating between absorption at the interoptode distances of 3 and 4 cm. As determined by such spatial resolution, the SmO$_2$ was little, if at all, influenced by cutaneous and subcutaneous blood flow.[17] In muscle, ~75% of blood is in venules or veins, and the INVOS 4100 spectrophotometer has been calibrated against a tissue oxygen saturation in arterial (25% of the signal) and internal jugular vein (75% of the signal) blood. With spatially resolved dual-wavelength NIRS of skeletal muscle, 100% saturation refers to total oxygenation of hemoglobin and myoglobin, as myoglobin attenuates near-infrared light with an absorption spectrum comparable with that of hemoglobin. The muscle NIRS measurements were made in 12 control subjects and in 14 patients with type 2 diabetes.

**Statistical analysis**

Data analysis comparing patients with type 2 diabetes and control subjects was performed using the Student's t test and correlations between data sets were determined using the Pearson correlation coefficient. Data are presented as means $\pm$ standard error of the mean (SEM). Statistical significance was taken at $p < 0.05$.

**Results**

**Patient characteristics and echocardiography results**

There were no significant differences in sex, age or body mass index between the patients with type 2 diabetes and the control subjects (Table 1).

Table 1. *Patient characteristics and echocardiography parameters, and fasting blood metabolite concentrations, in control subjects (n=15) and patients with type 2 diabetes (n=21).*

|  | Control subjects | Diabetic patients |
|---|---|---|
| Number of males (% of n) | 11(73%) | 15(71%) |
| Age (y) | 52±3 | 57±2 |
| Body mass index (BMI, kg.m$^{-2}$) | 25.2±0.4 | 28.6±0.5 |
| Diabetes duration (y) | - | 3.3±0.6 |
| Systolic blood pressure (mmHg) | 126±5 | 133±3 |
| Diastolic blood pressure (mmHg) | 76±2 | 74±2 |
| Mean heart rate (beats.min$^{-1}$) | 70±5 | 68±2 |
| LVESD (cm) | 2.8±0.2 | 3.0±0.2 |
| LVEDD (cm) | 4.3±0.2 | 4.9±0.2 |
| IVSD (cm) | 0.8±0.1 | 1.1±0.1 |
| LVMI (g.m$^{-2}$) | 141±19 | 141±10 |
| E/A | 1.29±0.05 | 0.98±0.12 |
| EF | 0.60±0.02 | 0.61±0.06 |
| HbA$_1$c (%) | 5.7±0.1 | 8.3±0.4*** |
| Glucose (mmol.l$^{-1}$) | 5.1±0.1 | 9.5±0.6*** |
| Free fatty acids (mmol.l$^{-1}$) | 0.40±0.05 | 0.55±0.04* |
| Lactate (mmol.l$^{-1}$) | 1.1±0.2 | 1.5±0.1* |
| Cholesterol (mmol.l$^{-1}$) | 4.9±0.2 | 4.7±0.3 |
| Triglycerides (mmol.l$^{-1}$) | 1.4±0.2 | 1.8±0.2 |
| HDL cholesterol (mmol.l$^{-1}$) | 1.3±0.1 | 1.1±0.1 |

Data are expressed as means±SEM. LVESD, left ventricular end-systolic diameter; LVEDD, left ventricular end-diastolic diameter; IVSD, interventricular septum diameter; LVMI, left ventricular mass index; E/A, early flow velocity to late atrial contraction ratio; EF, ejection fraction. HbA$_1$c, glycosylated hemoglobin; HDL, high density lipoprotein. *, $p < 0.05$; ***, $p < 0.001$ vs. control. Mean duration of type 2 diabetes was 3.3±0.6 years from the time of diagnosis. Systolic and diastolic blood pressures and heart rates were similar in the two groups. Echocardiography showed normal left ventricular systolic and diastolic function in patients with no abnormalities in left ventricular chamber thickness or diameter, or any other parameter (Table 1). The patients with diabetes had no history of cardiovascular disease and no clinical signs of impaired cardiac or skeletal muscle blood flow.

**Blood parameters**

Fasting blood HbA$_1$c and glucose levels were 1.5-fold and 1.9-fold higher, respectively, in patients with type 2 diabetes than in controls (Table 1). Plasma levels of free fatty acids were 1.4-fold higher in diabetic patients, as were lactate levels. Total cholesterol, triglycerides and HDL cholesterol were formal in the patients with diabetes.

**Cardiac high energy phosphate metabolism**

Figure1 shows typical examples of cardiac $^{31}$P MR spectra from a normal subject (PCr/ATP=2.35) and a patient with type 2 diabetes (PCr/ATP=1.35). The mean cardiac PCr/ATP ratio was $2.30\pm0.12$ in control subjects, but was decreased by 35%, to $1.50\pm0.11$ (p<0.001), in patients with diabetes. The PCr/ATP ratios correlated negatively with the plasma free fatty acid concentrations in all subjects ($r^2$=0.32; p<0.01; Figure 2), and positively with fasting plasma glucose concentrations in the diabetic patients ($r^2$=0.55; p<0.05; Figure 2), but there were no correlations with plasma lactate or HbA$_{1c}$ levels. The PDE/ATP ratios were the same in the controls ($0.51\pm0.06$) and the diabetic patients ($0.51\pm0.12$), as were the chemical shift differences between the $\alpha$- and $\beta$-phosphate peaks of ATP, being $8.3\pm0.4$ and $8.5\pm0.1$ ppm for controls and diabetic patients, respectively.

**Skeletal muscle high energy phosphate metabolism**

We found that the average exercise times for the patients with diabetes were 32% shorter, at 7 min, compared with the control subjects at 11 min (Table 2 and Figure 3).

Table 2. *Skeletal muscle energy metabolites, pH and oxygenation at rest, during exercise and at the end of exercise in control subjects and patients with type 2 diabetes.*

| | Control subjects | | Diabetic patients | |
|---|---|---|---|---|
| | *Rest* | *End-exercise* | *Rest* | *End-exercise* |
| PCr (mmol.l$^{-1}$) | $34\pm1$ | $17\pm2$ | $35\pm1$ | $18\pm2$ |
| Pi (mmol.l$^{-1}$) | $4.6\pm0.2$ | - | $4.7\pm0.1$ | - |
| pH | $7.04\pm0.01$ | $6.90\pm0.04$ | $7.04\pm0.01$ | $6.84\pm0.05$ |
| ADP ($\mu$mol.l$^{-1}$) | $15\pm2$ | $60\pm6$ | $11\pm1$ | $57\pm8$ |
| $\delta(\alpha$-$\beta)$ ATP (ppm) | $8.29\pm0.01$ | $8.31\pm0.07$ | $8.28\pm0.01$ | $8.40\pm0.07$ |
| Oxygen saturation (%) | $68\pm3$ | $57\pm3$ | $71\pm2$ | $60\pm3$ |

| | Control subjects | Diabetic patients |
|---|---|---|
| | *Exercise* | |
| Exercise times (min) | $10.5\pm0.6$ | $7.1\pm0.6$*** |
| PCr hydrolysis (mmol.l$^{-1}$.min$^{-1}$) | $1.6\pm0.1$ | $3.2\pm0.6$* |
| pH decline (pH units.min$^{-1}$) | $0.013\pm0.003$ | $0.036\pm0.009$* |
| Free ADP production ($\mu$mol.l$^{-1}$.min$^{-1}$) | $4.2\pm0.5$ | $9.5\pm3.4$ |
| Tissue deoxygenation (%.min$^{-1}$) | $0.8\pm0.3$ | $2.5\pm0.4$** |
| | *Recovery* | |
| Initial PCr formation (mmol.l$^{-1}$.min$^{-1}$) | $20\pm2$ | $15\pm2$* |
| PCr recovery half-time (s) | $32\pm3$ | $52\pm7$* |
| Free ADP recovery half-times (s) | $18\pm6$ | $19\pm2$ |
| Reoxygenation time (s) | $56\pm10$ | $140\pm18$*** |

Data are expressed as means$\pm$SEM. ADP, adenosine diphosphate; PCr, phosphocreatine; Pi, inorganic phosphate; $\delta(\alpha$-$\beta)$ATP, chemical shift differences between the $\alpha$- and $\beta$-phosphate peaks of ATP. *, p<0.05; **, p<0.01; ***, p<0.001 vs. control.

Figure 4 shows examples of skeletal muscle spectra before and at the end of the standardised exercise protocol in a patient with type 2 diabetes and at the equivalent time (5.1 min) of exercise in a control subject. Under resting conditions, skeletal muscle pH and PCr (PCr/ATP), free ADP and inorganic phosphate concentrations were the same in controls and patients with type 2 diabetes (Table 2). During exercise, PCr hydrolysis was 2-fold faster and the pH decrease was 3-fold faster in the patients with diabetes compared with the control subjects, but the free ADP production rates were not significantly different (Table 2). In all subjects, fatigue occurred when PCr depletion was ~50% ($50\pm4$% in controls vs. $51\pm4$% in diabetics) and at the same pH and free ADP concentrations (Table 2). The free magnesium concentrations remained unaltered during exercise in all subjects (Table 2). Following exercise, the initial rate of PCr recovery was 25% slower and the PCr recovery half-times were 1.6-fold longer in patients with type 2 diabetes than in controls, but the free ADP recovery half-times were the same (Table 2).

The exercise times correlated negatively with the $HbA_{1c}$ levels ($r^2$=0.32; p<0.01; Figure 5) and the plasma glucose levels ($r^2$=0.23; p<0.01; correlation not shown), but there were no correlations with the plasma free fatty acid or lactate levels. The rates of PCr hydrolysis and pH decrease during exercise did not correlate with any of the fasting metabolite concentrations. However, the PCr recovery half-times correlated positively with the $HbA_{1c}$ levels ($r^2$=0.40; p<0.001; correlation not shown) and the plasma glucose concentrations ($r^2$=0.16; p<0.05; correlation not shown) for all subjects, but there were no correlations with the plasma free fatty acid or lactate concentrations.

**Skeletal muscle oxygenation**

At rest, gastrocnemius muscle oxygen saturation was stable and the same for both groups, 68% in controls and 71 % in diabetics, and all subjects stopped exercising after an 11% decrease in tissue oxygenation measured using NIRS (Table 2). The first diabetic patient stopped exercising after 3 min (Figure 4), therefore, during the first 3 min of exercise, the rate of deoxygenation was 3.1-fold faster in the type 2 diabetic patients than in the controls (Table 2), and correlated with exercise time ($r^2$=0.29, p<0.01, Figure 5). Similarly, the reoxygenation times during recovery after exercise were 2.5 times longer in patients with diabetes compared with controls (Table 2), correlating with the $HbA_{1c}$ levels ($r^2$=0.35; p<0.01; Figure 5) and with PCr recovery half-times ($r^2$=0.25; p<0.01; Figure 5) in all subjects, but not with the plasma free fatty acid or lactate levels.

The above results show that increases in free fatty acids, associated with type 2 diabetes can contribute to muscle impairment, particularly cardiac muscle impairment. These findings are also relevant to other disorders/conditions associated with high levels of free fatty acids, and so reduction of free fatty acids may be a general aim in reducing the likelihood of muscle impairment, e.g. heart failure.

**References**

1. Schernthaner G. Cardiovascular mortality and morbidity in type-2 diabetes mellitus. Diabetes Res Clin Pract. 1996;31:S3-S13.

2. Taegtmeyer H, McNulty P, Young ME. Adaptation and maladaptation of the heart in diabetes: Part I. General concepts. Circulation. 2002;105:1727-1733.

3. Iribarren C, Karter AJ, Go AS, et al. Glycemic control and heart failure among adult patients with diabetes. Circulation. 2001;103:2668-2673.

4. Rodrigues B, Cam MC, McNeill JH. Metabolic disturbances in diabetic cardiomyopathy. Mol CellBiochem. 1998;180:53-57.

5. Chatham JC, SeymourA-ML. Cardiac carbohydrate metabolism in Zucker diabetic fatty rats. Cardiovasc Res. 2002;55:104-112.

6. Sidell RJ, Cole MA, Draper NJ, et al. Thiazolidinedione treatment normalizes insulin resistance and ischemic injury in the Zucker fatty rat heart. Diabetes. 2002;51:1110-1117.

7. Stanley WC, Lopaschuk GD, McCormack JG. Regulation of energy substrate metabolism in the diabetic heart. Cardiovasc Res. 1997;34:25-33.

8. Tune JD, Yeh C, Setty S, et al. Coronary blood flow control is impaired at rest and during exercise in conscious diabetic dogs. Basic Res Cardiol. 2002;97:248-257.

9. Meyer C, Schwaiger M. Myocardial blood flow and glucose metabolism in diabetes mellitus. Am J Cardiol. 1997;80:94A-101A.

10. lozzo P, Chareonthaitawee P, Rimoldi O, et al. Mismatch between insulin-mediated glucose uptake and blood flow in the heart of patients with Type II diabetes. Diabetologia. 2002;45:1404-1409.

11. Baron AD, Laakso M, Brechtel G, et al. Mechanism of insulin resistance in insulin-dependent diabetes mellitus: A major role for reduced skeletal muscle blood flow. J Clin Endocrinol Metab. 1991;73:637-643.

12. Demir I, Ermis C, Altunbas H, et al. Serum HbA1c levels and exercise capacity in diabetic patients. Jpn Heart J. 2001;42:607-616.

13. Estacio RO, Regensteiner JG, Wolfel EE, et al. The association between diabetic complications and exercise capacity in NIDDM patients. Diabetes Care. 1998;21:291-295.

14. Crilley JG, Boehm EA, Rajagopalan B, et al. Magnetic resonance spectroscopy evidence of abnormal cardiac energetics in Xp21 muscular dystrophy. J Am Coll Cardiol. 2000;36:1953-1958.

15. Lodi R, Kemp GJ, Muntoni F, et al. Reduced cytosolic acidification during exercise suggests defective glycolytic activity in skeletal muscle of patients with Becker muscular dystrophy. An in vivo 31P magnetic resonance spectroscopy study. Brain. 1999;122:121-130.

16. Veech RL, Lawson JWR, Cornell NW, et al. Cytosolic phosphorylation potential. J Biol Chem.

1979;254:6538-6547.

17. Madsen PL, Secher NH. Near-infrared oximetry of the brain. Prog Neurobiol. 1999; 58:541-560.

18. Neubauer S, Horn M, Cramer M, et al. Myocardial phosphocreatine-to-ATP ratio is a predictor of mortality in patients with dilated cardiomyopathy. Circulation. 1997;96:2190-2196.

19. Boehm EA, Jones BE, Radda GK, et al. Increased uncoupling proteins and decreased efficiency in the palmitate-perfused hyperthyroid rat heart. Am J Physiol. 2001;280: H977-H983.

20. Chatham JC, Gao ZP, Bonen A, et al. Preferential inhibition of lactate oxidation relative to glucose oxidation in the rat heart following diabetes. Cardiovasc Res. 1999;43:96-106.

21. Libby P, Plutzky J. Diabetic macrovascular disease. The glucose paradox? Circulation. 2002;106:2760-2763.

22. Mahler RJ, Adler ML. Type 2 diabetes mellitus: update on diagnosis, pathophysiology, and treatment. J Clin Endocrinol Metab. 1999;84:1165-1171.

23. Komiyama T, Shigematsu H, Yasuhara H, et al. Near-infrared spectroscopy grades the severity of intermittent claudication in diabetes more accurately than ankle pressure measurement. Br J Surg. 2000; 87:459-466.

## Claims

1. A compound for use in the therapeutic treatment of muscle impairment of the human or animal body by administering a compound to a subject that reduces free fatty acids circulating in the blood plasma of the subject, the compound comprising a ketone body ester.

2. A compound for use in the therapeutic treatment of muscle impairment according to claim 1, wherein the subject has diabetes.

3. A compound for use in the therapeutic treatment of muscle impairment according to claim 2, wherein the subject has Type 2 diabetes.

4. A compound for use in the treatment or prevention of muscle impairment or fatigue according to any one of the preceding claims, wherein the compound is for the treatment or prevention of cardiac muscle fatigue or skeletal muscle fatigue.

5. A compound for use in the treatment or prevention of muscle impairment or fatigue according to any one of the preceding claims, wherein the compound is in the form of a food supplement or liquid composition.

6. Use of a compound that reduces free fatty acids circulating in the blood plasma of a subject, the compound comprising a ketone body ester in the non-therapeutic treatment of muscle impairment or fatigue.

7. Use according to claim 6 wherein the compound is for the non-therapeutic treatment of cardiac muscle fatigue or skeletal muscle fatigue.

8. Use according to claim 6 or claim 7 wherein the compound is in the form of a food supplement or liquid composition.

9. A liquid composition for rehydration during or after exercise, comprising water, a sugar carbohydrate and a compound that reduces free fatty acids circulating in blood plasma the compound being a ketone body ester.

10. A composition according to claim 9, wherein the sugar carbohydrate is glucose.

## Patentansprüche

1. Eine Verbindung zur Verwendung bei der therapeutischen Behandlung von Muskelbeeinträchtigung des menschlichen oder tierischen Körpers, wobei einem Betroffenem eine Verbindung verabreicht wird, die freie, im Blutplasma des Betroffenen zirkulierende Fettsäuren reduziert, wobei die Verbindung einen Ketonkörperester umfasst.

2. Eine Verbindung zur Verwendung bei der therapeutischen Behandlung von Muskelbeeinträchtigung nach Anspruch 1, wobei der Betroffene Diabetes hat.

3. Eine Verbindung zur Verwendung bei der therapeutischen Behandlung von Muskelbeeinträchtigung nach Anspruch 2, wobei der Betroffene Diabetes Typ 2 hat.

4. Eine Verbindung zur Verwendung bei der Behandlung oder Vorbeugung von Muskelbeeinträchtigung oder -ermüdung nach einem der vorherigen Ansprüche, wobei die Verbindung eingesetzt wird zur Behandlung oder Vorbeugung von Herzmuskelermüdung oder Skelettmuskelermüdung.

5. Eine Verbindung zur Verwendung in der Behandlung oder Vorbeugung von Muskelbeeinträchtigung oder -ermüdung nach einem der vorherigen Ansprüche, wobei die Verbindung vorliegt in Form eines Nahrungsmittelzusatzes oder einer flüssigen Zusammensetzung.

6. Verwendung einer Verbindung, die im Blutplasma des Betroffenen zirkulierende Fettsäuren reduziert, in der nichttherapeutischen Behandlung von Muskelbeeinträchtigung oder -ermüdung, wobei die Verbindung einen Ketonkörperester umfasst.

7. Verwendung nach Anspruch 6, wobei die Verbindung für die nichttherapeutische Behandlung von Herzmuskelermüdung oder Skelettmuskelermüdung ist.

8. Verwendung nach Anspruch 6 oder 7, wobei die Verbindung in Form eines Nahrungsmittelzusatzes oder als flüssige Zusammensetzung vorliegt.

9. Eine flüssige Zusammensetzung zur Rehydratation während oder nach einer Übung, umfassend Wasser, ein Zucker-Kohlenhydrat und eine Verbindung, die freie, im Blutplasma zirkulierende Fettsäuren reduziert, wobei die Verbindung ein Ketonkörperester ist.

10. Eine Zusammensetzung nach Anspruch 9, wobei das Zucker-Kohlenhydrat Glukose ist.


**Revendications**

1. Composé destiné à être utilisé pour le traitement thérapeutique d'une insuffisance musculaire du corps humain ou animal en administrant à un sujet un composé qui réduit la circulation d'acides gras libres dans le plasma sanguin du sujet, le composé comprenant un ester de corps cétonique.

2. Composé destiné à être utilisé pour le traitement thérapeutique d'une insuffisance musculaire selon la revendication 1, dans lequel le sujet est atteint de diabète.

3. Composé destiné à être utilisé pour le traitement thérapeutique d'une insuffisance musculaire selon la revendication 2, dans lequel le sujet est atteint de diabète du type 2.

4. Composé destiné à être utilisé pour le traitement ou la prévention d'une insuffisance ou fatigue musculaire selon l'une quelconque des revendications précédentes, dans lequel le composé est destiné à assurer le traitement ou la prévention de la fatigue musculaire cardiaque ou de la fatigue musculaire squelettique.

5. Composé destiné à être utilisé pour le traitement ou la prévention d'une insuffisance ou fatigue musculaire selon l'une quelconque des revendications précédentes, dans lequel le composé est sous la forme d'un complément alimentaire ou d'une composition liquide.

6. Utilisation d'un composé qui réduit la circulation d'acides gras libres circulant dans le plasma sanguin d'un sujet, le composé comprenant un ester de corps cétonique, pour le traitement non thérapeutique de l'insuffisance ou de la fatigue musculaire.

7. Utilisation selon la revendication 6, dans laquelle le composé est destiné au traitement non thérapeutique de la fatigue musculaire cardiaque ou de la fatigue musculaire squelettique.

8. Utilisation selon la revendication 6 ou 7, dans laquelle le composé est sous la forme d'un complément alimentaire ou d'une composition liquide.

9. Composition liquide destinée à la réhydratation pendant ou après l'exercice, comprenant de l'eau, un sucre à base d'hydrates de carbone et un composé qui réduit la circulation des acides gras libres dans le plasma sanguin, le composé étant un ester de corps cétonique.

10. Composition selon la revendication 9, dans laquelle le sucre à base d'hydrates de carbone est du glucose.

Figure 1

Cardiac ³¹P MR spectra

Figure 2

Figure 3

Figure 4

Figure 5

**EP 1 641 444 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0064876 A
- WO 0174834 A
- WO 02028857 A
- WO 9924451 A
- WO 0151645 A
- WO A0064876 A

**Non-patent literature cited in the description**

- **SCHERNTHANER G.** Cardiovascular mortality and morbidity in type-2 diabetes mellitus. *Diabetes Res Clin Pract.,* 1996, vol. 31, S3-S13
- **TAEGTMEYER H ; MCNULTY P ; YOUNG ME.** Adaptation and maladaptation of the heart in diabetes: Part I. General concepts. *Circulation,* 2002, vol. 105, 1727-1733
- **IRIBARREN C ; KARTER AJ ; GO AS et al.** Glycemic control and heart failure among adult patients with diabetes. *Circulation,* 2001, vol. 103, 2668-2673
- **RODRIGUES B ; CAM MC ; MCNEILL JH.** Metabolic disturbances in diabetic cardiomyopathy. *Mol Cell-Biochem.,* 1998, vol. 180, 53-57
- **CHATHAM JC ; SEYMOURA-ML.** Cardiac carbohydrate metabolism in Zucker diabetic fatty rats. *Cardiovasc Res.,* 2002, vol. 55, 104-112
- **SIDELL RJ ; COLE MA ; DRAPER NJ et al.** Thiazolidinedione treatment normalizes insulin resistance and ischemic injury in the Zucker fatty rat heart. *Diabetes,* 2002, vol. 51, 1110-1117
- **STANLEY WC ; LOPASCHUK GD ; MCCORMACK JG.** Regulation of energy substrate metabolism in the diabetic heart. *Cardiovasc Res.,* 1997, vol. 34, 25-33
- **TUNE JD ; YEH C ; SETTY S et al.** Coronary blood flow control is impaired at rest and during exercise in conscious diabetic dogs. *Basic Res Cardiol.,* 2002, vol. 97, 248-257
- **MEYER C ; SCHWAIGER M.** Myocardial blood flow and glucose metabolism in diabetes mellitus. *Am J Cardiol.,* 1997, vol. 80, 94A-101A
- **LOZZO P ; CHAREONTHAITAWEE P ; RIMOLDI O et al.** Mismatch between insulin-mediated glucose uptake and blood flow in the heart of patients with Type II diabetes. *Diabetologia,* 2002, vol. 45, 1404-1409
- **BARON AD ; LAAKSO M ; BRECHTEL G et al.** Mechanism of insulin resistance in insulin-dependent diabetes mellitus: A major role for reduced skeletal muscle blood flow. *J Clin Endocrinol Metab.,* 1991, vol. 73, 637-643
- **DEMIR I ; ERMIS C ; ALTUNBAS H et al.** Serum HbA1c levels and exercise capacity in diabetic patients. *Jpn Heart J.,* 2001, vol. 42, 607-616
- **ESTACIO RO ; REGENSTEINER JG ; WOLFEL EE et al.** The association between diabetic complications and exercise capacity in NIDDM patients. *Diabetes Care,* 1998, vol. 21, 291-295
- **CRILLEY JG ; BOEHM EA ; RAJAGOPALAN B et al.** Magnetic resonance spectroscopy evidence of abnormal cardiac energetics in Xp21 muscular dystrophy. *J Am Coll Cardiol,* 2000, vol. 36, 1953-1958
- **LODI R ; KEMP GJ ; MUNTONI F et al.** Reduced cytosolic acidification during exercise suggests defective glycolytic activity in skeletal muscle of patients with Becker muscular dystrophy. An in vivo P magnetic resonance spectroscopy study. *Brain,* 1999, vol. 122, 121-130
- **VEECH RL ; LAWSON JWR ; CORNELL NW et al.** Cytosolic phosphorylation potential. *J Biol Chem.,* 1979, vol. 254, 6538-6547
- **MADSEN PL ; SECHER NH.** Near-infrared oximetry of the brain. *Prog Neurobiol.,* 1999, vol. 58, 541-560
- **NEUBAUER S ; HORN M, CRAMER M et al.** Myocardial phosphocreatine-to-ATP ratio is a predictor of mortality in patients with dilated cardiomyopathy. *Circulation,* 1997, vol. 96, 2190-2196
- **BOEHM EA ; JONES BE ; RADDA GK et al.** Increased uncoupling proteins and decreased efficiency in the palmitate-perfused hyperthyroid rat heart. *Am J Physiol.,* 2001, vol. 280, H977-H983
- **CHATHAM JC ; GAO ZP ; BONEN A et al.** Preferential inhibition of lactate oxidation relative to glucose oxidation in the rat heart following diabetes. *Cardiovasc Res.,* 1999, vol. 43, 96-106
- **LIBBY P ; PLUTZKY J.** Diabetic macrovascular disease. The glucose paradox?. *Circulation,* 2002, vol. 106, 2760-2763

- **MAHLER RJ ; ADLER ML.** Type 2 diabetes mellitus: update on diagnosis, pathophysiology, and treatment. *J Clin Endocrinol Metab.,* 1999, vol. 84, 1165-1171

- **KOMIYAMA T ; SHIGEMATSU H ; YASUHARA H et al.** Near-infrared spectroscopy grades the severity of intermittent claudication in diabetes more accurately than ankle pressure measurement. *Br J Surg.,* 2000, vol. 87, 459-466